# EUROPÄISCHE PATENTSCHRIFT

(11) **EP 2 670 446 B1**
(45) Veröffentlichungstag und Bekanntmachung des Hinweises auf die Patenterteilung: **12.10.2016**
(21) Anmeldenummer: 12702510.4
(22) Anmeldetag: 01.02.2012
(51) Int. Cl.: A61L 9/12, A61L 9/14, B60H 3/00

(54) **FUNKTIONSKOPF FÜR EINEN DUFTSTOFFBEHÄLTER**
FUNCTIONAL HEAD FOR A FRAGRANCE CONTAINER
TÊTE FONCTIONNELLE POUR RÉCIPIENT CONTENANT UNE SUBSTANCE ODORANTE

(30) Priorität: 04.02.2011 DE 102011010277
(43) Veröffentlichungstag der Anmeldung: 11.12.2013
(73) Patentinhaber: Leopold Kostal GmbH & Co. KG, 58513 Lüdenscheid (DE)
(72) Erfinder: FEHLING, André, 44265 Dortmund (DE)
(74) Vertreter: Kerkmann, Detlef
(86) Internationale Anmeldenummer: PCT/EP2012/051698
(87) Internationale Veröffentlichungsnummer: WO 2012/104351

(56) Entgegenhaltungen:
- EP-A2- 0 361 437
- WO-A1-00/01421
- WO-A1-2011/101384

## Beschreibung

Die Erfindung betrifft einen Funktionskopf für einen Duftstoffbehälter, mit einer Einlassöffnung zum Einlass von Luft in einen Einlasskanal, mit einer Auslassöffnung zum Auslass eines Duftstoff-Luft-Gemisches aus einem Auslasskanal, und mit einer federbelasteten Ventilplatte, welche den Einlasskanal und den Auslasskanal beim Anfügen des Funktionskopfs an einen Duftgenerator öffnet und im vom Duftgenerator getrennten Zustand geschlossen hält.

WO 2011/101384, Stand der Technik unter Art. 54(3) EPÜ, offenbart ein Gerät zur Duftausbringung in einem Kraftfahrzeug, mit einem Duftstoffbehälter und mit einer elektrischen Einrichtung zur Erzeugung eines Luftstroms zur Verteilung eines in dem Duftstoffbehälter enthaltenen Duftstoffs, mit einem Gehäuse, welches die elektrische Einrichtung enthält, und das einen Adapter aufweist, an den der Duftstoffbehälter anfügbar ist, wobei der Duftstoffbehälter eine Ventilkappe aufweist, die ein federbelastetes Ventil ausbildet, das beim Anfügen an den Adapter geöffnet wird und den Duftstoffbehälter strömungsleitend mit der elektrischen Einrichtung verbindet. WO 00/01421 offenbart ein Verfahren zum Beseitigen oder Neutralisieren von Gerüchen in der Luft,wobei Flüssigkeitströpfchen aus einer Sprühvorrichtung, die eine Geruchsbeseitigungszusammensetzung enthält, auf die Geruchsquelle gerichtet werden, wobei das Verfahren das Übertrageneiner unipolaren Ladung auf die Flüssigkeitströpfchen mittels Doppelschichtladungwährend des Versprühens der Flüssigkeitströpfchen durch die Sprühvorrichtung aufweist.

Ein derartiger Funktionskopf für einen Duftstoffbehälter ist in der nicht vorveröffentlichten deutschen Patentanmeldung DE 10 2010 008 436 beschrieben. Der Duftstoffbehälter ist ein Flakon, auf dessen Hals der Funktionskopf in Form einer Ventilkappe aufschraubbar ist. Der Funktionskopf weist auf seiner Oberseite eine mit einer Feder belastete Ventilplatte auf. Der Duftstoffbehälter mit der aufgeschraubten Ventilkappe ist über eine Ankoppelvorrichtung an ein elektrisches Gerät zur Duftausbringung, das hier kurz als Duftgenerator bezeichnet ist und das vorzugsweise in einem Kraftfahrzeug verwendet wird, anfügbar. Beim Anfügevorgang wird die Ventilplatte gegen die Kraft der Feder verschoben, wodurch die Ventilplatte einen Einlasskanal für Luft und einen Auslasskanal für ein Duftstoff-Luft-Gemisch freigibt und mit der Ankoppelvorrichtung verbindet, die einen Luftstrom in den Einlasskanal einleitet und die das Duftstoff-Luft-Gemisch aus dem Auslasskanal, beispielsweise in den Innenraum eines Kraftfahrzeug, ausleitet.

Wird der Duftstoffbehälter mit dem Funktionskopf von der Ankoppelvorrichtung getrennt, so verschließt die federbelastete Ventilplatte automatisch die beiden Kanäle. Allerdings können durch eine Druckeinwirkung auf die Ventilplatte die beiden Kanäle ohne weiteres wieder geöffnet werden, wobei die Gefahr besteht, dass der flüssige Duftstoff aus dem Duftstoffbehälter austritt. In der Patentanmeldung DE 10 2010 008 436 wird daher vorgeschlagen, bei einem der Ankoppelvorrichtung entnommenen Duftstoffbehälter den Funktionskopf mit einer Schutzkappe zu versehen, die eine versehentliche Druckeinwirkung auf die Ventilplatte verhindert.

Nachteilig hierbei ist, dass mitunter die Schutzkappe bei der Entnahme des Duftstoffbehälters nicht zur Hand ist, so dass der Verwender gezwungen ist, den Duftstoffbehälter ohne die Schutzkappe zu belassen. Kann der Duftstoffbehälter nun nicht standsicher platziert werden, was besonders bei einer Verwendung in einem Kraftfahrzeug vorkommen kann, so besteht die Gefahr, dass die Ventilplatte in Kontakt mit anderen Gegenständen kommt und dabei eine Druckeinwirkung erfährt, wodurch die Ventilplatte den Ein- und den Auslasskanal freigibt. Hierdurch kann Duftstoff in konzentrierter flüssiger Form austreten. Da Duftstoffe naturgemäß sehr geruchsintensive Substanzen sind, können so unangenehme Kontaminationen des Fahrzeuginnenraums entstehen, die, wenn überhaupt, nur mit erheblichem Aufwand zu beseitigen sind.

Es stellte sich die Aufgabe, einen Funktionskopf für einen Duftstoffbehälter zu schaffen, der einen unbeabsichtigten Austritt eines Duftstoffs und eine daraus resultierenden Duftstoffkontaminationen mit hoher Sicherheit verhindert. Diese Aufgabe wird erfindungsgemäß dadurch gelöst, dass die Einlassöffnung und die Auslassöffnung in einem Gehäusewandabschnitt des Funktionskopfs angeordnet sind, dass die Ventilplatte im Inneren des Funktionskopfs hinter dem Gehäusewandabschnitt federbelastet angeordnet ist und in einer Ventilposition die Einlassöffnung und die Auslassöffnung verschließt, und dass der Gehäusewandabschnitt des Funktionskopfs eine Ausnehmung aufweist, in die ein Stößel einschiebbar ist, durch den die Ventilplatte gegen die Kraft der Feder verschiebbar ist.

Der Funktionskopf ist erfindungsgemäß so ausgebildet, dass die Ventilplatte bei Entnahme des Duftstoffsbehälters von der Ankoppelvorrichtung des Duftgenerators die Einlassöffnung und die Auslassöffnung automatisch verschließt und zudem nicht durch eine unbeabsichtigte Einwirkung von außen betätigbar ist. Dies wird dadurch erreicht, dass die Ventilplatte im Inneren des Funktionskopfs angeordnet ist, und von dort die Einlassöffnung und die Auslassöffnung abdichtet. Hierdurch ist die Ventilplatte vor einer versehentlichen Betätigung geschützt. Auf die Ventilplatte kann lediglich über eine Ausnehmung im Gehäusewandabschnitt des Funktionskopfes zugegriffen werden, wobei zum Verschieben der Ventilplatte durch die Ausnehmung hindurch ein dünner stiftförmiger Gegenstand erforderlich ist, der hier als Stößel bezeichnet wird. Ein derart geformter Stößel ist zweckmäßigerweise fest oder durch einen Aktuator beweglich an der Ankoppelvorrichtung angeordnet. Form und Größe der Ausnehmung im Funktionskopf können so ausgebildet sein, dass ein unbeabsichtigtes Eindringen eines stiftartigen Gegenstand praktisch ausgeschlossen werden kann.

Weitere vorteilhafte Ausgestaltungen und Weiterbildungen der Erfindung sind in den abhängigen Ansprüchen beschrieben.

So kann der Funktionskopf vorteilhaft eine auf den Duftstoffbehälter aufschraubbare Kappe ausbilden. Besonders vorteilhaft ist es, wenn der Funktionskopf mit dem Duftstoffbehälter, beispielsweise durch einen umgebördelten Kragenabschnitt, untrennbar verbunden ist. Hierdurch ist auch ein versehentliches Lösen der Verbindung zwischen dem Funktionskopf und dem Duftstoffbehälter ausgeschlossen.

Ebenfalls vorteilhaft ist es, wenn der Einlass- und/oder der Auslasskanal jeweils ein Labyrinthsystem ausbildet, so dass im Duftstoffbehälter bzw. im Funktionskopf schwappender Duftstoff nicht bis zu der Einlassbeziehungsweise der Auslassöffnung gelangen kann. Das Labyrinthsystem kann dabei vorteilhaft so ausgebildet sein, dass die Ventilplatte außer der Einlass- und der Auslassöffnung noch jeweils eine weitere, innerhalb des Funktionskopfs gelegene Ventilöffnung des Einlasskanals und/oder des Auslasskanals verschließt. Hierdurch wird eine besonders sichere Abdichtung erreicht.

Der Funktionskopf kann vorteilhafterweise hinsichtlich des Einlass- und des Auslasskanals einen symmetrischen Aufbau aufweisen, wodurch die Konstruktion des Funktionskopfs vereinfacht wird. Vorteilhaft ist es auch, am Funktionskopf Rastelemente zur Herstellung einer Rastverbindung zum Duftgenerator vorzusehen.

Der Funktionskopf ist besonders vorteilhaft als Bestandteil eines Beduftungssystems in einem Kraftfahrzeug einsetzbar.

Im Folgenden wird der Aufbau und die Funktionsweise der Erfindung anhand eines in der Zeichnung schematisch dargestellten Ausführungsbeispiels erläutert. Es zeigen
Figur 1 eine Schnittansicht entlang der Einlassöffnung des Funktionskopfs,
Figur 2 eine Schnittansicht entlang der Auslassöffnung des Funktionskopfs,
Figur 3 eine Draufsicht auf den Funktionskopf,
Figur 4 eine Ventilplatte als Einzelteil,
Figur 5 eine Schnittansicht mit einer die Einlass- bzw. Auslassöffnung abdichtende Ventilplatte.

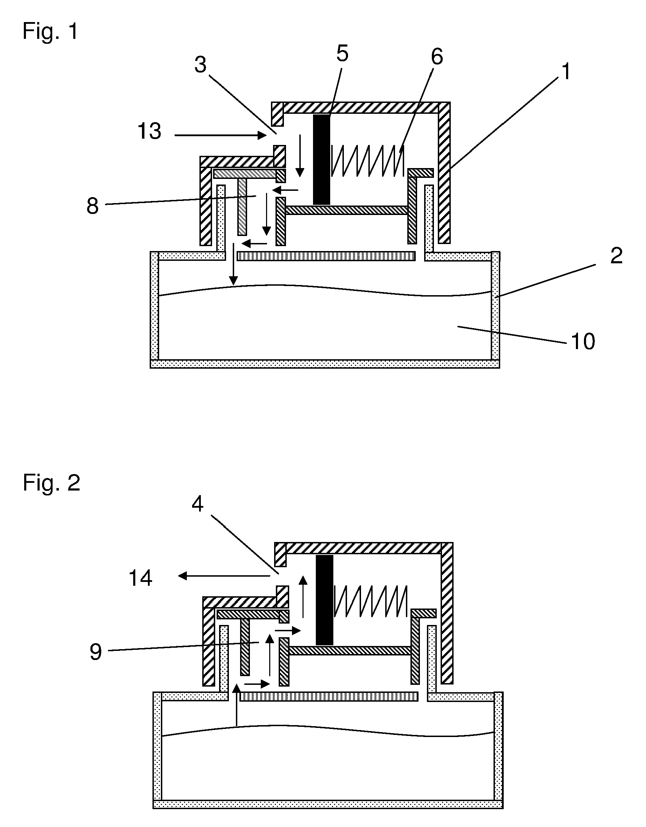

Die Figur 1 zeigt skizzenhaft einen Schnitt durch einen Funktionskopf 1 und einen mit dem Funktionskopf 1 flüssigkeitsdicht verbundenen Duftstoffbehälter 2. Der Funktionskopf 1 ist entweder mit dem Hals des Duftstoffbehälters 2 verschraubt oder vorzugsweise auf eine andere Weise untrennbar mit dem Duftstoffbehälter 2 verbunden. Die skizzierte Anordnung kann an einen hier nicht dargestellten Duftgenerator angefügt sein, und Bestandteil eines Beduftungssystems sein, welches beispielsweise in einem Kraftfahrzeug verwendet wird.

Die in der Figur 1 dargestellte Schnittebene verläuft durch die Einlassöffnung 3 des Funktionskopfes 1. In einem Beduftungssystem wird von dem hier nicht dargestellten Duftgenerator Luft über die Einlassöffnung 3 und einen damit verbundenen Einlasskanal 8 in den mit einem Duftstoff 10 befüllten Duftstoffbehälter 2 eingeleitet. Der Weg des in den Funktionskopf 1 eintretenden Luftstroms 13 von der Einlassöffnung 3 durch den labyrinthartig gestalteten, das heiß mit mehreren Abwinklungen versehenen, Einlasskanal 8 in den Duftstoffbehälter 2 ist durch eine Abfolge von Richtungspfeilen angedeutet.

Die mit Molekülen des Duftstoffs 10 angereicherte Luft gelangt über einen Auslasskanal 9 und eine Auslassöffnung 4 zum Duftgenerator und wird von diesem in den Innenraum des Kraftfahrzeugs ausgeleitet. Die Figur 2 verdeutlicht den Weg des austretenden Luftstroms 14 wiederum durch eine Reihe von Richtungspfeilen. Wie der Vergleich der Figuren 1 und 2 zeigt, können der Einlass- und der Auslassbereich des Funktionskopfs 1 symmetrisch oder sogar vollkommen gleichartig ausgestaltet sein, wodurch der Aufwand zur Konstruktion des Funktionskopfs 1 gering gehalten werden kann.

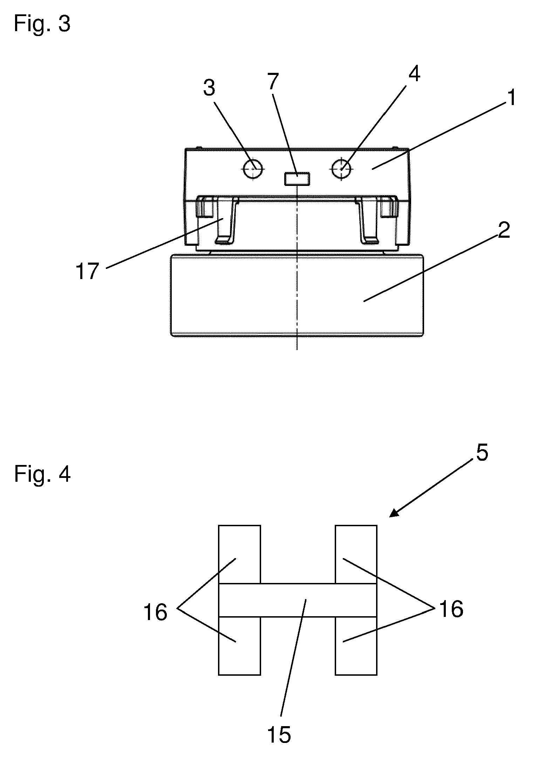

Die Figur 3 zeigt eine Ansicht von außen auf den Funktionskopf 1 und den damit verbundenen Duftstoffbehälter 2. Erkennbar ist die Einlassöffnung 3 und die Auslassöffnung 4. Dazwischen befindet sich als weitere Ausnehmung eine Stößelöffnung 7. Durch die Stößelöffnung 7 ist der Zugriff auf den Ansteuerbereich 15 der Ventilplatte 5 möglich, welche in der Figur 4 als Einzelteil skizziert ist. An senkrecht vom Ansteuerbereich 15 abstehenden Abschnitten weist die Ventilplatte 5 insgesamt vier Dichtbereiche 16 auf, welche zum flüssigkeitsdichten Schließen und Öffnen der Einlass- und Auslassöffnungen 3, 4 sowie von weiteren Ventilöffnungen 11 geeignet ausgebildet sind.

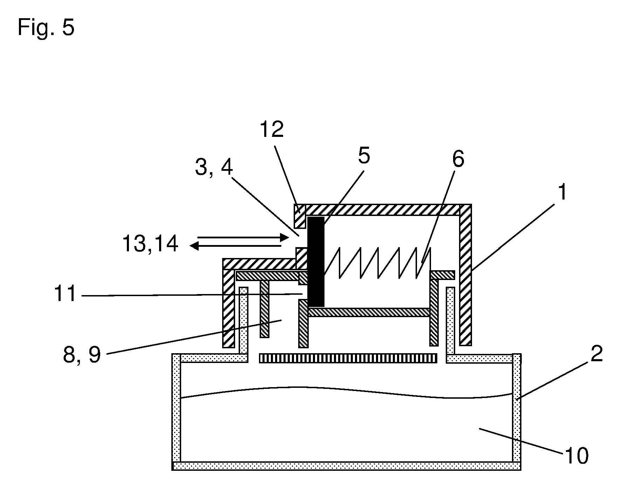

Greift kein Stößel, der zweckmäßigerweise Bestandteil einer am Duftgenerator vorgesehenen Ankoppelvorrichtung ist, in die Stößelöffnung 7 ein, so drückt die Feder 6 die Ventilplatte 5 gegen die Innenseite des Gehäusewandabschnitts 12, was in der Figur 5 dargestellt ist. Hierdurch verschließt die Ventilplatte 5 sowohl die Einlassöffnung 3 als auch die Auslassöffnung 4. Aufgrund des symmetrischen Aufbaus des Einlass- und Auslassbereichs des Funktionskopfs 1 werden diese eigentlich räumlich nebeneinander realisierten Ventilfunktionen hier durch eine einzige Skizze verdeutlicht.

Wie die Figur 5 des weiteren zeigt, dichtet die am Gehäusewandabschnitt 12 anliegende Ventilplatte 5, außer der Einlass- bzw. der Auslassöffnung 3, 4, jeweils eine weitere Ventilöffnung 11 ab, die sich durch den labyrinthartigen Aufbau des Einlass- bzw. Auslasskanals 8 bzw. 9 ergibt.

Die Ventilplatte 5 bildet somit innerhalb des Funktionskopfs 1 sowohl auf der Seite des Einlasskanals 8 als auch auf der Seite des Auslasskanals 9 jeweils ein Doppelventil aus, das erstens die Ein- und Auslassöffnungen 3, 4 abdichtet und dadurch den Funktionskopf 1 vor dem Eindringen von Verschmutzungen schützt und zweitens den Duftstoffbehälter 2 abdichtet, um ein Austreten des flüssigen Duftstoffs 10 zu verhindern.

Um das Beduftungssystem in den betriebsbereiten Zustand zu versetzen wird der Funktionskopf 1 mit der Ankoppelvorrichtung des Duftgenerators verbunden und mittels am Funktionskopf 1 angeformter Rastelemente 17 an diesem befestigt. Gleichzeitig verbinden sich nicht dargestellte Verbindungselemente des Duftgenerators mit der Einlassöffnung 3 und der Auslassöffnung 4. Beim Anfügen des Funktionskopfs 1 an den Duftgenerator greift der am Duftgenerator angeordnete Stößel in die Stößelöffnung 7 ein und öffnet durch Verschieben der Ventilplatte 5 gegen die Kraft der Feder 6 die Einlass- und Auslassöffnungen 3, 4, die Zugänge zu den Einlass- und Auslasskanälen 8, 9 sowie die internen Ventilöffnungen 11.

### Bezugszeichen

- 1: Funktionskopf
- 2: Duftstoffbehälter
- 3: Einlassöffnung
- 4: Auslassöffnung
- 5: Ventilplatte
- 6: Feder
- 7: Ausnehmung (Stößelöffnung)
- 8: Einlasskanal
- 9: Auslasskanal
- 10: Duftstoff
- 11: Ventilöffnung(en)
- 12: Gehäusewandabschnitt
- 13: Luftstrom
- 14: Luftstrom
- 15: Ansteuerbereich
- 16: Dichtbereiche
- 17: Rastelemente

## Patentansprüche

1. Funktionskopf (1) für einen Duftstoffbehälter (2),
mit einer Einlassöffnung (3) zum Einlass von Luft in einen Einlasskanal (8), mit einer Auslassöffnung (4) zum Auslass eines Duftstoff-Luft-Gemisches aus einem Auslasskanal (9), und
mit einer federbelasteten Ventilplatte (5), welche den Einlasskanal (8) und den Auslasskanal (9) beim Anfügen des Funktionskopfs (1) an einen Duftgenerator öffnet und im vom Duftgenerator getrennten Zustand geschlossen hält,
wobei
die Einlassöffnung (3) und die Auslassöffnung (4) in einem Gehäusewandabschnitt (12) des Funktionskopfs (1) angeordnet sind,
**dadurch gekennzeichnet,**
**dass** die Ventilplatte (5) im Inneren des Funktionskopfs (1) hinter dem Gehäusewandabschnitt (12) federbelastet angeordnet ist und in einer Ventilposition die Einlassöffnung (3) und die Auslassöffnung (4) verschließt, und
**dass** der Gehäusewandabschnitt (12) des Funktionskopfs (1) eine Ausnehmung (7) aufweist, in die ein Stößel einschiebbar ist, durch den die Ventilplatte (5) gegen die Kraft der Feder (6) verschiebbar ist.

2. Funktionskopf nach Anspruch 1, **dadurch gekennzeichnet, dass** der Funktionskopf (1) eine auf den Duftstoffbehälter (2) aufschraubbare Kappe ausbildet.

3. Funktionskopf nach Anspruch 1, **dadurch gekennzeichnet, dass** der Funktionskopf (1) mit dem Duftstoffbehälter (2) untrennbar verbindbar ist.

4. Funktionskopf nach Anspruch 1, **dadurch gekennzeichnet, dass** der Einlass- und der Auslasskanal (8, 9) jeweils mehrfach abgewinkelt ausgebildet sind.

5. Funktionskopf nach Anspruch 1, **dadurch gekennzeichnet, dass** der Funktionskopf (1) hinsichtlich des Einlass- und des Auslasskanals (8, 9) einen symmetrischen Aufbau aufweist.

6. Funktionskopf nach Anspruch 1, **dadurch gekennzeichnet, dass** die Ventilplatte (5) außer der Einlass- und der Auslassöffnung (3, 4) noch jeweils eine weitere, innerhalb des Einlasskanals (7) und/oder des Auslasskanals (8) gelegene Ventilöffnung (11) verschließt.

7. Funktionskopf nach Anspruch 1, **dadurch gekennzeichnet, dass** der Funktionskopf (1) Rastelemente (17) zur Herstellung einer Rastverbindung mit dem Duftgenerator aufweist.

8. Funktionskopf nach Anspruch 1, **dadurch gekennzeichnet, dass** der Funktionskopf (1) zur Verwendung bei einem Beduftungssystem in einem Kraftfahrzeug vorgesehen ist.

## Claims

1. Functional head (1) for a fragrance container (2)
having an inlet aperture (3) for admitting air into an inlet channel (8), having an outlet aperture (4) for discharging a fragrance-air mixture from an outlet channel (9), and
having a spring-loaded valve plate (5) which opens the inlet channel (8) and the outlet channel (9) upon attaching the functional head (1) to a fragrance generator and keeps it closed in a state separate from the fragrance generator,
whereby
the inlet aperture (3) and the outlet aperture (4) are located in a section of the housing wall (12) of the functional head (1),
**characterised in that**
the valve plate (5) is located in a spring-loaded manner behind the section of the housing wall (12) in the interior of the functional head (1) and closes the inlet aperture (3) and the outlet aperture (4) in one valve position,
and
that the section of the housing wall (12) of the functional head (1) shows a recess (7) into which a plunger can be inserted, as a result of which the valve plate (5) is displaceable against the force of the spring (6).

2. Functional head according to Claim 1, **characterised in that** the functional head (1) forms a cap that can be screwed onto the fragrance container (2).

3. Functional head according to Claim 1, **characterised in that** the functional head (1) can be inseparably attached to the fragrance container (2).

4. Functional head according to Claim 1, **characterised in that** the inlet and the outlet channel (8, 9) are each of a multi-angled design.

5. Functional head according to Claim 1, **characterised in that** the functional head (1) shows a symmetrical construction as regards the inlet and the outlet channel (8, 9).

6. Functional head according to Claim 1, **characterised in that**, besides the inlet and the outlet apertures (3, 4), the valve plate (5) also closes a further valve opening (11) positioned inside the inlet channel (7) and/or the outlet channel (8) in each case.

7. Functional head according to Claim 1, **characterised in that** the functional head (1) shows latching elements (17) for establishing a snap-on connection to the fragrance generator.

8. Functional head according to Claim 1, **characterised in that** the functional head (1) is provided for using for a scenting system in a motor vehicle.

## Revendications

1. Tête fonctionnelle (1) pour un récipient contenant une substance odoriférante (2), avec une ouverture d'entrée (3) pour l'introduction d'air dans un canal d'entrée (8), avec une ouverture de sortie (4) pour la sortie d'un mélange substance odoriférante-air par un canal de sortie (9), avec une plaque de soupape (5) commandée par ressort, qui ouvre le canal d'entrée (8) et le canal de sortie (9) lors du raccordement de la tête fonctionnelle (1) à un générateur de fragrance et les maintient fermés à l'état séparé du générateur d'odeur, sachant que l'ouverture d'entrée (3) et l'ouverture de sortie (4) sont disposées, commandées par ressort, dans une section de paroi de boîtier (12) de la tête fonctionnelle (1),
**caractérisée en ce que**
la plaque de soupape (5) est disposée, commandée par ressort, à l'intérieur de la tête fonctionnelle (1), derrière la section de paroi de boîtier (12), et ferme, dans une position de soupape, l'ouverture d'entrée (3) et l'ouverture de sortie (4),
et que
la section de paroi de boîtier (12) de la tête fonctionnelle (1) présente un évidement (7), dans lequel peut être introduit un poussoir au moyen duquel la plaque de soupape (5) peut être déplacée contre la force du ressort (6).

2. Tête fonctionnelle selon la revendication 1, **caractérisée en ce que** la tête fonctionnelle (1) forme un capuchon, qui peut être vissé sur le récipient contenant une substance odoriférante (2).

3. Tête fonctionnelle selon la revendication 1, **caractérisée en ce que** la tête fonctionnelle (1) peut être reliée inséparablement au récipient contenant une substance odoriférante (2).

4. Tête fonctionnelle selon la revendication 1, **caractérisée en ce que** le canal d'entrée et le canal de sortie (8,9) sont chacun de conception plusieurs fois coudée.

5. Tête fonctionnelle selon la revendication 1, **caractérisée en ce que** la tête fonctionnelle (1) présente une construction symétrique en ce qui concerne le canal d'entrée et le canal de sortie (8,9).

6. Tête fonctionnelle selon la revendication 1, **caractérisée en ce que**, outre l'ouverture d'entrée et l'ouverture de sortie (3,4), la plaque de soupape (5), ferme aussi, respectivement, une autre ouverture de soupape (11), située à l'intérieur du canal d'entrée (7) et / ou du canal de sortie (8).

7. Tête fonctionnelle selon la revendication 1, **caractérisée en ce que** la tête fonctionnelle (1) présente des éléments d'encliquetage (17) pour la réalisation d'un encliquetage sur le générateur de fragrance.

8. Tête fonctionnelle selon la revendication 1, **caractérisée en ce que** la tête fonctionnelle (1) est prévue pour un système de diffusion de fragrance dans un véhicule automobile.
